# EUROPÄISCHE PATENTSCHRIFT

(11) **EP 1 230 200 B1**
(45) Veröffentlichungstag und Bekanntmachung des Hinweises auf die Patenterteilung: **10.03.2004**
(21) Anmeldenummer: 00987265.6
(22) Anmeldetag: 17.11.2000
(51) Int. Cl.: C07C 2/10, C07C 11/02, C07C 45/50, C07C 47/02, C07C 29/16, C07C 31/125, C07C 43/11, C07F 9/11, C07F 9/09, C11D 1/34

(54) **C13-ALKOHOLGEMISCH UND FUNKTIONALISIERTES C13-ALKOHOLGEMISCH**
C13 ALCOHOL MIXTURE AND FUNCTIONALISED C13 ALCOHOL MIXTURE
MELANGE D'ALCOOL C13 ET MELANGE D'ALCOOL C13 FONCTIONNALISE

(30) Priorität: 18.11.1999 DE 19955593
(43) Veröffentlichungstag der Anmeldung: 14.08.2002
(73) Patentinhaber: BASF AKTIENGESELLSCHAFT, 67056 Ludwigshafen (DE)
(72) Erfinder: ZELLER, Edgar, 68165 Mannheim (DE); WALTER, Marc, 67227 Frankenthal (DE); RICHTER, Wolfgang, 67157 Wachenheim (DE); DIEHL, Klaus, 67454 Hassloch (DE); RÖPER, Michael, 67157 Wachenheim (DE); TROPSCH, Jürgen, 67354 Römerberg (DE)
(74) Vertreter: Kinzebach, Werner, Dr.
(86) Internationale Anmeldenummer: PCT/EP2000/011440
(87) Internationale Veröffentlichungsnummer: WO 2001/036356

(56) Entgegenhaltungen:
- DE-A- 4 339 713
- GB-A- 1 471 481

## Beschreibung

Die vorliegende Erfindung betrifft ein C₁₃-Alkoholgemisch, das sich insbesondere zur Herstellung von Tensiden eignet. Sie betrifft weiter ein funktionalisiertes C₁₃-Alkoholgemisch mit Tensideigenschaften.

Es ist bekannt, Fettalkohole mit etwa 8 bis 20 Kohlenstoffatomen zur Herstellung von nichtionischen und anionischen Tensiden einzusetzen. Dazu werden die Alkohole einer entsprechenden Funktionalisierung, z. B. durch Alkoxylierung oder Glycosidierung, unterworfen. Die erhaltenen Alkoxylate können entweder direkt als nichtionische oberflächenaktive Substanzen eingesetzt oder durch eine weitere Funktionalisierung, z. B. durch Sulfatierung oder Phosphatierung, in anionische oberflächenaktive Substanzen überführt werden. Die anwendungstechnischen Eigenschaften dieser Tenside, z. B. deren Netzvermögen, Schaumbildung, Fettlösevermögen, biologische Abbaubarkeit usw., werden im Wesentlichen durch die Kettenlänge und den Verzweigungsgrad des hydrophoben Kohlenwasserstoffrestes des eingesetzten Alkohols bestimmt. Alkohole, die sich gut zur Weiterverarbeitung zu wirksamen Tensiden eignen, werden auch als Tensidalkohole bezeichnet.

In Kosswig/Stache, "Die Tenside", Carl Hanser Verlag, München, Wien, 1993, Kapitel 2.2 und 2.3, ist die Umsetzung von Fettalkoholen mit Alkylenoxiden zu den entsprechenden Fettalkoholalkoxylaten sowie deren Sulfatierung und Phosphatierung beschrieben.

Fettalkohole sind sowohl aus nativen Quellen als auch auf synthetischem Weg, z. B. durch Aufbau aus Edukten mit einer geringeren Zahl an Kohlenstoffatomen erhältlich. So erhält man z. B. nach dem SHOP-Prozess (Shell Higher Olefine Process) ausgehend von Ethen Olefinfraktionen mit einer für die Weiterverarbeitung zu Tensiden geeigneten Kohlenstoffanzahl. Die Funktionalisierung der Olefine zu den entsprechenden Alkoholen erfolgt dabei z. B. durch Hydroformylierung und Hydrierung. Nachteilig an den auf Ethylen basierenden Verfahren zur Herstellung von Fettalkoholen sind die hohen Kosten des Ausgangsmaterials, wodurch diese Verfahren wirtschaftlich benachteiligt sind.

Olefine mit einer zur Weiterverarbeitung zu Tensidalkoholen geeigneten Kohlenstoffzahl können auch durch Oligomerisation von C₃-C₆-Alkenen, wie insbesondere Propen oder Buten oder Gemischen davon, erhalten werden.

Beim DIMERSOL-Prozess (vergleiche Revue de l'Institut Français du Petrole, Vol. 37, No. 5, Sept./Okt. 1982, S. 639ff) werden Propen oder Buten in homogener Phase in Gegenwart eines Katalysatorsystems aus einem Übergangsmetallderivat und einer metallorganischen Verbindung oligomerisiert. Typische Katalysatorsysteme sind Ni(O)-Komplexe in Verbindung mit Lewis-Säuren wie AlCl₃, BF₃, SbF₅ usw. oder Ni(II)-Komplexe in Verbindung mit Alkylaluminiumhalogeniden. Ein Nachteil dieses homogenkatalytischen Verfahrens besteht in der aufwendigen Abtrennung des Katalysators vom Reaktionsgemisch. Ein weiterer Nachteil liegt darin, dass die erhaltenen höheren Olefine stets mit Halogenspuren verunreinigt sind, die vom Oligomerisierungskatalysator herrühren und bei dessen Abtrennung nicht vollständig entfernt werden.

Der Restgehalt an Halogen verursacht eine beschleunigte Korrosion der Anlagenteile, die mit dem Produktstrom in Berührung kommen. Außerdem beeinträchtigen Halogenspuren in den höheren Olefinen die Aktivität der Katalysatoren in den Weiterverarbeitungsstufen, insbesondere des Hydroformylierungs- und Hydrierkatalysators. Die Halogenspuren liegen überwiegend in Form halogen-organischer Verbindungen vor. Das Vorliegen derartiger Verbindungen in Produkten, die zu Consumer-Produkten weiterverarbeitet werden, ist aus grundsätzlichen Erwägungen unerwünscht.

Es ist weiter bekannt, niedere Olefine mittels heterogener saurer Katalysatoren, z. B. geträgerter Phosphorsäure, zu oligomerisieren. Die Anwendung dieser heterogenen sauren Katalysatoren führt jedoch nachteiligerweise zu stark verzweigten höheren Olefinen.

Die WO 98/23566 beschreibt ein Gemisch verzweigter primärer Alkohole, das unter anderem durch Dimerisierung eines C₆-C₁₀-Olefins in Gegenwart eines homogenen Dimerisierungskatalysators und anschließende Umwandlung des erhaltenen verzweigten C₁₂-C₂₀-Olefins zum Gemisch verzweigter primärer Alkohole erhältlich ist.

Die anwendungstechnischen Eigenschaften der bekannten Tensidalkohole bzw. der daraus hergestellten funktionalisierten Tensidalkohole können nur bedingt befriedigen.

Der vorliegenden Erfindung liegt die Aufgabe zugrunde, halogenfreie Tensidalkohole und daraus gewonnene Tenside zur Verfügung zu stellen. Die Verzweigungsstruktur der Tensidalkohole soll derart sein, dass ein ausgewogenes Eigenschaftsspektrum hinsichtlich oberflächenaktiver Eigenschaften, Ökotoxizität und biologischer Abbaubarkeit der Tenside gegeben ist.

Erfindungsgemäß wird diese Aufgabe durch ein Verfahren zur Herstellung eines C₁₃-Alkoholgemischs gelöst, bei dem man
a) einen Butene enthaltenden C₄-Kohlenwasserstoffstrom, der weniger als 5 Gew.-%, bezogen auf die Butenfraktion, iso-Buten enthält, bei erhöhter Temperatur mit einem Nickel enthaltenden heterogenen Katalysator in Kontakt bringt,
b) aus dem Reaktionsgemisch eine C₁₂-Olefinfraktion isoliert,
c) die C₁₂-Olefinfraktion durch Umsetzung mit Kohlenmonoxid und Wasserstoff in Gegenwart eines Kobalt-Katalysators hydroformyliert und
d) hydriert.

Die Erfindung betrifft außerdem ein C₁₃-Alkoholgemisch, das nach dem vorstehenden Verfahren erhältlich ist.

Das erfindungsgemäße C₁₃-Alkoholgemisch ist vorzugsweise im Wesentlichen halogenfrei, d. h. es enthält weniger als 3 Gew.-ppm, insbesondere weniger als 1 Gew.-ppm, Halogen, insbesondere Chlor.

### a) Butentrimerisierung

Zur Herstellung des C₁₃-Alkoholgemischs werden in einem ersten Schritt C₁₂-Olefine aus C₄-Körpern aufgebaut. Hierzu werden Butene in an sich bekannter Weise an einem Nickel enthaltenden heterogenen Katalysator oligomerisiert. In Abhängigkeit von den gewählten Verfahrensbedingungen werden unterschiedliche relative Mengen an Buten-Dimeren, -Trimeren und höheren Oligomeren erhalten. Für die vorliegenden Zwecke werden die Buten-Trimere, d. h. C₁₂-Olefine, weiter verarbeitet. Im Hinblick auf den gewünschten Verzweigungsgrad des nach Hydroformylierung/Hydrierung erhaltenen C₁₃-Alkoholgemisches müssen die eingesetzten Butene überwiegend linear sein, d. h. der Gehalt an iso-Butenen soll weniger als 5 Gew.-% betragen. Die Butene können eine Beimischung gesättigter C₄-Kohlenwasserstoffe enthalten, die als Verdünnungsmittel bei der Oligomerisierung wirken.

Die verwendbaren heterogenen, Nickel enthaltenden Katalysatoren können unterschiedliche Strukturen aufweisen, wobei Nickeloxid enthaltende Katalysatoren bevorzugt sind. Es kommen an sich bekannte Katalysatoren in Betracht, wie sie in C. T. O'Connor et al., Catalysis Today, Bd. 6 (1990), S. 336-338 beschrieben sind. Insbesondere werden trägergebundene Nickelkatalysatoren eingesetzt. Die Trägermaterialien können z. B. Kieselsäure, Tonerde, Aluminosilicate, Aluminosilicate mit Schichtstrukturen und Zeolithe, wie Mordenit, Faujasit, Zeolith X, Zeolith-Y und ZSM-5, Zirkoniumoxid, das mit Säuren behandelt ist, oder sulfatiertes Titandioxid sein. Besonders geeignet sind Fällungskatalysatoren, die durch Mischen wässriger Lösungen von Nickelsalzen und Silicaten, z. B. Natriumsilicat mit Nickelnitrat, und gegebenenfalls Aluminiumsalzen, wie Aluminiumnitrat, und Calcinieren erhältlich sind. Weiterhin sind Katalysatoren verwendbar, die durch Einlagerung von Ni²⁺-Ionen durch Ionenaustausch in natürliche oder synthetische Schichtsilicate, wie Montmorillonite, erhalten werden. Geeignete Katalysatoren können auch durch Imprägnieren von Kieselsäure, Tonerde oder Alumosilicaten mit wässrigen Lösungen löslicher Nickelsalze, wie Nickelnitrat, Nickelsulfat oder Nickelchlorid, und anschließende Calcinierung erhalten werden.

Besonders bevorzugt sind Katalysatoren, die im Wesentlichen aus NiO, SiO₂, TiO₂ und/oder ZrO₂ sowie gegebenenfalls Al₂O₃ bestehen. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält. Ein solcher Katalysator ist durch Fällung der Katalysatormasse bei pH 5 bis 9 durch Zugabe einer Nickelnitrat enthaltenden wässrigen Lösung zu einer Alkaliwasserglaslösung, die Titandioxid und/oder Zirkondioxid enthält, Filtrieren, Trocknen und Tempern bei 350 bis 650 °C erhältlich. Zur Herstellung dieser Katalysatoren wird im einzelnen auf die DE-4339713 verwiesen. Auf die Offenbarung dieser Druckschrift und den darin zitierten Stand der Technik wird vollinhaltlich Bezug genommen.

Der Katalysator liegt vorzugsweise in stückiger Form, z. B. in Form von Tabletten, z. B. mit einem Durchmesser von 2 bis 6 mm und einer Höhe von 3 bis 5 mm, Ringen mit z. B. 5 bis 7 mm Außendurchmesser, 2 bis 5 mm Höhe und 2 bis 3 mm Lochdurchmesser, oder Strängen unterschiedlicher Länge eines Durchmessers von z. B. 1,5 bis 5 mm, vor. Derartige Formen werden auf an sich bekannte Weise durch Tablettierung oder Extrusion, meist unter Verwendung eines Tablettierhilfsmittels, wie Graphit oder Stearinsäure, erhalten.

Der C₄-Kohlenwasserstoffstrom enthält in der Regel 50 bis 100 Gew.-%, vorzugsweise 60 bis 90 Gew.-%, Butene und 0 bis 50 Gew.-%, vorzugsweise 10 bis 40 Gew.-%, Butane. Die Butenfraktion umfasst weniger als 5 Gew.-%, insbesondere weniger als 3 Gew.-% Isobuten, bezogen auf die Butenfraktion. Die Butenfraktion weist im Allgemeinen folgende Zusammensetzung auf (jeweils bezogen auf die Butenfraktion):

| | |
|---|---|
| 1-Buten | 1 bis 50 Gew.-% |
| cis-2-Buten | 1 bis 50 Gew.-% |
| trans-2-Buten | 1 bis 99 Gew.-% |
| iso-Buten | 1 bis 5 Gew.-% |

Als besonders bevorzugter Einsatzstoff wird das sogenannte Raffinat II verwendet, bei dem es sich um einen iso-Butenen-abgereicherten C₄-Schnitt aus einer FCC-Anlage oder einem Steamcracker handelt.

Sind Diolefine oder Alkine im C₄-Kohlenwasserstoffstrom vorhanden, so werden diese vor der Oligomerisierung vorzugsweise auf weniger als 10 Gew.-ppm, insbesondere weniger als 5 Gew.-ppm, besonders bevorzugt weniger als 1 Gew.-ppm aus demselben entfernt. Sie werden bevorzugt durch selektive Hydrierung, z. B. gemäß EP-81041 und DE-1568542 entfernt.

Aus dem olefinreichen Kohlenwasserstoffgemisch werden zweckmäßigerweise außerdem sauerstoffhaltige Verbindungen, wie Alkohole, Aldehyde, Ketone oder Ether, weitgehend entfernt. Hierzu kann der C₄-Kohlenwasserstoffstrom mit Vorteil über ein Adsorptionsmittel, wie z. B. ein Molekularsieb, insbesondere eines mit einem Porendurchmesser von > 4 Å bis 5 Å, geleitet werden. Die Konzentration an sauerstoffhaltigen Verbindungen im C₄-Kohlenwasserstoffstrom beträgt vorzugsweise weniger als 1 Gew.-ppm, insbesondere weniger als 0,5 Gew.-ppm.

Das Inkontaktbringen mit dem Oligomerisierungskatalysator erfolgt vorzugsweise bei Temperaturen von 30 bis 280 °C, insbesondere 30 bis 140 °C und besonders bevorzugt von 40 bis 130 °C. Es erfolgt vorzugsweise bei einem Druck von 10 bis 300 bar, insbesondere von 15 bis 100 bar und besonders bevorzugt von 20 bis 80 bar. Der Druck wird dabei zweckmäßigerweise so eingestellt, dass bei der gewählten Temperatur der C₄-Kohlenwasserstoffstrom flüssig oder im überkritischen Zustand vorliegt.

Üblicherweise wird der C₄-Kohlenwasserstoffstrom über ein oder mehrere fest angeordnete Katalysatorbetten geleitet. Geeignete gegebenenfalls druckfeste Reaktionsapparaturen für das Inkontaktbringen des Kohlenwasserstoffstromes mit dem heterogenen Katalysator sind dem Fachmann bekannt. Dazu zählen.die allgemein üblichen Reaktoren für Gas/Fest-Reaktionen bzw. Flüssig/Fest-Reaktionen. Geeignet sind z. B. Rohrbündelreaktoren oder Schachtöfen.

Aufgrund der geringeren Investitionskosten sind Schachtöfen bevorzugt. Die Oligomerisierung kann in einem einzelnen Reaktor durchgeführt werden, wobei der Oligomerisierungskatalysator in einem einzigen oder mehreren Festbetten im Reaktor angeordnet sein kann. Alternativ kann eine Reaktorkaskade aus mehreren, vorzugsweise zwei, hintereinandergeschalteten Reaktoren eingesetzt werden, wobei beim Passieren des bzw. der dem letzten Reaktor der Kaskade vorgeschalteten Reaktors bzw. Reaktoren die Oligomerisierung der Butene nur bis zu einem Teilumsatz betrieben wird und der gewünschte Endumsatz erst beim Passieren des Reaktionsgemisches durch den letzten Reaktor der Kaskade erzielt wird. In den einzelnen Reaktoren der Reaktorkaskade kann der Oligomerisierungskatalysator in einem einzigen oder in mehreren Katalysatorbetten angeordnet sein. Weiterhin können in den einzelnen Reaktoren der Reaktorkaskade unterschiedliche Reaktionsbedingungen hinsichtlich Druck und/oder Temperatur im Rahmen der oben genannten Druck- und Temperaturbereiche eingestellt werden. Außerdem ist es möglich, in den einzelnen Reaktoren der Kaskade unterschiedliche Oligomerisierungskatalysatoren einzusetzen, obgleich die Anwendung des gleichen Katalysators in sämtlichen Reaktoren der Kaskade bevorzugt ist. Bei dem bevorzugten Reaktor handelt es sich in der Regel um ein mit dem Katalysator beschicktes, vertikales zylindrisches Rohr, das von dem olefinreichen Kohlenwasserstoffgemisch z. B. von oben nach unten durchströmt wird.

Nach dem Verlassen des Reaktors bzw. des letzten Reaktors einer Kaskade werden aus dem Reaktoraustrag die gebildeten Oligomere von den nicht umgesetzten Butenen und Butanen abgetrennt. Die gebildeten Oligomere können in einem nachfolgenden Vakuumfraktionierungsschritt aufgetrennt bzw. aufgereinigt werden.

Bisweilen kann es zweckmäßig sein, den von den gebildeten Oligomeren befreite Reaktoraustrag, der im Wesentlichen aus nicht umgesetzten Butenen und Butanen besteht, vollständig oder zum Teil zurückzuführen.

### b) Isolierung einer C₁₂-Olefinfraktion

Aus dem Reaktionsaustrag der Oligomerisierungsreaktion wird in einem oder mehreren Trennschritten eine C₁₂-Olefinfraktion isoliert. Geeignete Trennvorrichtungen sind die üblichen, dem Fachmann bekannten Apparaturen. Dazu zählen z. B. Destillationskolonnen, wie Bodenkolonnen, die gewünschtenfalls mit Glocken, Siebplatten, Siebböden, Ventilen, Seitenabzügen usw. ausgerüstet sein können, Verdampfer, wie Dünnschichtverdampfer, Fallfilmverdampfer, Wischblattverdampfer, Sambay-Verdampfer usw. und Kombinationen davon. Bevorzugt erfolgt die Isolierung der C₁₂-Olefinfraktion durch fraktionierte Destillation.

Der ISO-Index der C₁₂-Olefinfraktion, der die mittlere Zahl der Verzweigungen angibt, beträgt in der Regel 1,9 bis 2,3, vorzugsweise 2,0 bis 2,3. Der ISO-Index kann z. B. ermittelt werden, indem eine Probe der C₁₂-Olefinfraktion zu den Dodecanen hydriert wird und im ¹H-NMR-Spektrum anhand der den Methylgruppen zuzuordnenden Signalfläche und der den Gesamtprotonen zuzuordnenden Signalfläche die mittlere Anzahl der Methylgruppen bestimmt wird. Der ISO-Index ergibt sich als mittlere Zahl der Methylgruppen abzüglich zwei.

### c) Hydroformylierung

Zur Herstellung des erfindungsgemäßen Alkoholgemisches wird die isolierte C₁₂-Olefinfraktion zu C₁₃-Aldehyden hydroformyliert und anschließend zu C₁₃-Alkoholen hydriert. Dabei kann die Herstellung der Alkoholgemische einstufig oder in zwei separaten Reaktionsschritten erfolgen.

Eine Übersicht über Hydroformylierungsverfahren und geeignete Katalysatoren findet sich in Beller et al., Journal of Molecular Catalysis A 104 (1995), S. 17-85.

Es ist für die vorliegende Erfindung kritisch, dass die Hydroformylierung in Gegenwart eines Kobalt-Hydroformylierungskatalysators erfolgt. Die Menge des Hydroformylierungskatalysators beträgt im Allgemeinen 0,001 bis 0,5 Gew.-%, gerechnet als Kobaltmetall, bezogen auf die Menge der zu hydroformylierenden Olefine. Die Reaktionstemperatur liegt im Allgemeinen im Bereich von etwa 100 bis 250 °C, bevorzugt 150 bis 210 °C. Die Reaktion kann bei einem erhöhten Druck von etwa 10 bis 650 bar durchgeführt werden. Es ist bevorzugt, dass die Hydroformylierung in Gegenwart von Wasser erfolgt; sie kann allerdings auch in Abwesenheit von Wasser durchgeführt werden.

Kohlenmonoxid und Wasserstoff werden üblicherweise in Form eines Gemisches, dem sogenannten Synthesegas, eingesetzt. Die Zusammensetzung des eingesetzten Synthesegases kann in weiten Bereich variieren. Das molare Verhältnis von Kohlenmonoxid und Wasserstoff beträgt in der Regel etwa 2,5:1 bis 1:2,5. Ein bevorzugtes Verhältnis liegt bei etwa 1:1,5.

Der hydroformylierungsaktive Kobaltkatalysator ist HCo(CO)₄. Der Katalysator kann außerhalb des Hydroformylierungsreaktors, z. B. aus einem Kobalt(II)-salz in Gegenwart von Synthesegas, präformiert und zusammen mit den C₁₂-Olefinen und dem Synthesegas in den Hydroformylierungsreaktor eingeführt werden. Alternativ kann die Bildung der katalytisch aktiven Spezies aus Katalysatorvorstufen erst unter den Hydroformylierungsbedingungen, d. h in der Reaktionszone, erfolgen. Geeignete Katalysatorvorstufen sind Kobalt(II)-salze, wie Kobalt(II)-carboxylate, z. B. Kobalt(II)-formiat oder Kobalt(II)-acetat; sowie Kobalt(II)-acetylacetonat oder CO₂(CO)₈.

Der homogen im Reaktionsmedium gelöste Kobaltkatalysator kann vom Hydroformylierungsprodukt geeigneterweise abgetrennt werden, indem der Reaktionsaustrag der Hydroformylierung in Gegenwart einer sauren wässrigen Lösung mit Sauerstoff oder Luft behandelt wird. Dabei wird der Kobaltkatalysator unter Bildung von Kobalt(II)-salzen oxidativ zerstört. Die Kobalt(II)-salze sind wasserlöslich und werden in die wässrige Phase extrahiert, die abgetrennt und in das Hydroformylierungsverfahren zurückgeführt werden kann.

Zur kontinuierlichen Durchführung der Hydroformylierung kann man z. B. so vorgehen, dass (i) eine wässrige Kobalt(II)-salzlösung mit Wasserstoff und Kohlenmonoxid unter Bildung eines hydroformylierungsaktiven Kobaltkatalysators innig in Kontakt gebracht wird; (ii) die den Kobaltkatalysator enthaltende wässrige Phase in einer Reaktionszone mit den Olefinen sowie Wasserstoff und Kohlenmonoxid innig in Kontakt gebracht wird, wobei der Kobaltkatalysator in die organische Phase extrahiert wird und die Olefine hydroformyliert werden; und (iii) der Austrag aus der Reaktionszone mit Sauerstoff behandelt wird, wobei der Kobaltkatalysator unter Bildung von Kobalt(II)-salzen zersetzt wird, die Kobalt(II)-salze in die wässrige Phase zurückextrahiert werden und die Phasen getrennt werden. Die wässrige Kobalt(II)-salzlösung wird dann in das verfahren zurückgeführt. Als geeignete Kobalt(II)-salze kommen vor allem Kobalt(II)-acetat und Kobalt(II)-formiat in Betracht. Mit Vorteil kann die Bildung des Kobaltkatalysators, die Extraktion des Kobaltkatalysators in die organische Phase und die Hydroformylierung der Olefine in einem Schritt erfolgen, indem die wässrige Kobalt(II)-salzlösung, die Olefine und gegebenenfalls das organische Lösungsmittel sowie Wasserstoff und Kohlenmonoxid in der Reaktionszone unter Hydroformylierungsbedingungen, z. B. mittels einer Mischdüse, innig in Kontakt gebracht werden.

Die bei der Hydroformylierung erhaltenen rohen Aldehyde bzw. Aldehyd/Alkohol-Gemische können vor der Hydrierung gewünschtenfalls nach üblichen, dem Fachmann bekannten Verfahren isoliert und gegebenenfalls gereinigt werden.

### d) Hydrierung

Zur Hydrierung werden die bei der Hydroformylierung erhaltenen Reaktionsgemische mit Wasserstoff in Gegenwart eines Hydrierkatalysators umgesetzt.

Geeignete Hydrierkatalysatoren sind im Allgemeinen Übergangsmetalle, wie z. B. Cr, Mo, W, Fe, Rh, Co, Ni, Pd, Pt, Ru usw. oder deren Mischungen, die zur Erhöhung der Aktivität und Stabilität auf Trägern, wie z. B. Aktivkohle, Aluminiumoxid, Kieselgur usw. aufgebracht werden können. Zur Erhöhung der katalytischen Aktivität können Fe, Co und bevorzugt Ni, auch in Form der Raney-Katalysatoren als Metallschwamm mit einer sehr großen Oberfläche verwendet werden. Bevorzugt wird für die Herstellung der erfindungsgemäßen Tensidalkohole ein Co/Mo-Katalysator eingesetzt. Die Hydrierung der Oxo-Aldehyde erfolgt in Abhängigkeit von der Aktivität des Katalysators vorzugsweise bei erhöhten Temperaturen und erhöhtem Druck. Vorzugsweise liegt die Hydriertemperatur bei etwa 80 bis 250 °C, bevorzugt liegt der Druck bei etwa 50 bis 350 bar.

Aus dem nach der Hydrierung erhaltenen Reaktionsgemisch kann nach üblichen, dem Fachmann bekannten Reinigungsverfahren, insbesondere durch fraktionierte Destillation, das erfindungsgemäße C₁₃-Alkoholgemisch rein gewonnen werden.

Das erfindungsgemäße C₁₃-Alkoholgemisch weist vorzugsweise einen mittleren Verzweigungsgrad von 2,1 bis 2,5, insbesondere 2,2 bis 2,4 auf. Als Verzweigungsgrad ist die Zahl der Methylgruppen in einem Molekül des Alkohols abzüglich 1 definiert. Der mittlere verzweigungsgrad ist der statistische Mittelwert der Verzweigungsgrade der Moleküle einer Probe. Die mittlere Zahl der Methylgruppen in den Molekülen einer Probe kann leicht ¹H-NMR-spektroskopisch ermittelt werden. Hierzu wird die den Methylprotonen entsprechende Signalfläche im ¹H-NMR-Spektrum einer Probe durch drei dividiert und zu der durch zwei dividierten Signalfläche der Methylenprotonen in der CH₂-OH-Gruppe ins Verhältnis gesetzt.

Die Erfindung betrifft außerdem ein funktionalisiertes Alkoholgemisch, das dadurch erhalten wird, dass ein oben beschriebenes C₁₃-Alkoholgemisch einer
(i) Alkoxylierung,
(ii) Glycosidierung,
(iii) Sulfatierung,
(iv) Phosphatierung,
(v) Alkoxylierung und nachfolgender Sulfatierung, oder
(vi) Alkoxylierung und nachfolgender Phosphatierung
unterworfen wird.

Die Alkoxylierung der Alkoholgemische erfolgt durch Umsetzung mit mindestens einem Alkylenoxid. Bevorzugt sind die Alkylenoxide ausgewählt unter Verbindungen der allgemeinen Formel I 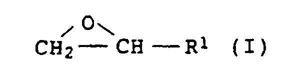 worin
R¹ für Wasserstoff oder einen geradkettigen oder verzweigten C₁- bis C₁₆-Alkylrest steht,
und Mischungen davon.

Bevorzugt steht der Rest R¹ in der Formel I für einen geradkettigen oder verzweigten C₁- bis C₈-Alkylrest, insbesondere C₁- bis C₄-Alkylrest.

Bevorzugt sind die Alkylenoxide ausgewählt unter Ethylenoxid, Propylenoxid, Butylenoxid und Mischungen davon.

Die Umsetzung der Alkoholgemische mit dem/den Alkylenoxid(en) erfolgt nach üblichen, dem Fachmann bekannten Verfahren und in dafür üblichen Apparaturen.

Die mittlere Kettenlänge der Polyetherketten der so funktionalisierten Alkoholgemische kann durch das Molmengenverhältnis von Alkohol zu Alkylenoxid bestimmmt werden. Bevorzugt werden alkoxyliert Alkoholgemische mit etwa 1 bis 200, bevorzugt etwa 1 bis 50, insbesondere 1 bis 10 Alkylenoxideinheiten hergestellt.

Die Alkoholgemische können gewünschtenfalls nur mit einem Alkylenoxid oder mit zwei oder mehreren verschiedenen Alkylenoxiden umgesetzt werden. Bei der Umsetzung der Alkoholgemische mit einem Gemisch aus zwei oder mehreren Alkylenoxiden enthalten die resultierenden Alkoxylate die Alkylenoxideinheiten im Wesentlichen statistisch verteilt. Werden die Alkylenoxide getrennt nacheinander eingesetzt, so resultieren Alkoxylate, die entsprechen der Zugabereihenfolge die Alkylenoxideinheiten in Form von Blöcken einpolymerisiert enthalten.

Die Alkoxylierung kann durch starke Basen, wie Alkalihydroxide und Erdalkalihydroxide, Brönstedsäuren oder Lewissäuren, wie AlCl₃, BF₃ etc. katalysiert werden.

Die Alkoxylierung erfolgt vorzugsweise bei Temperaturen im Bereich von etwa 80 bis 250 °C, bevorzugt etwa 100 bis 220 °C. Der Druck liegt vorzugsweise zwischen Umgebungsdruck und 600 bar. Gewünschtenfalls kann das Alkylenoxid eine Inertgasbeimischung, z. B. von etwa 5 bis 60 %, enthalten.

Die durch Alkoxylierung erhaltenen funktionalisierten Alkoholgemische zeigen eine sehr gute Oberflächenaktivität und können als nichtionische Tenside in einer Vielzahl von Anwendungsbereichen vorteilhaft eingesetzt werden, z. B. als Tensid, Dispergiermittel, Papierhilfsmittel, Schmutzlösungsmittel, Korrosionsinhibitor, Hilfsmittel für Dispersionen oder Inkrustierungsinhibitor.

Die Glycosidierung der Alkoholgemische erfolgt durch ein-, zwei- oder mehrfache Umsetzung der erfindungsgemäßen Alkoholgemische mit Mono-, Di- oder Polysacchariden. Die Umsetzung erfolgt nach üblichen, dem Fachmann bekannten Verfahren. Dazu zählt zum einen die säurekatalysierte Umsetzung unter Wasserentzug. Geeignete Säuren sind z. B. Mineralsäuren, wie HCl und H₂SO₄. Dabei werden in der Regel Oligosaccharide mit statistischer Kettenlängenverteilung erhalten. Vorzugsweise liegt der durchschnittliche Oligomerisierungsgrad bei 1 bis 3 Saccharidresten. Nach einem weiteren geeigneten Verfahren kann das Saccharid zunächst durch Umsetzung mit einem niedermolekularen C₁- bis C₈-Alkanol, wie z. B. Ethanol, Propanol oder Butanol, acetalisiert werden. Die Acetalisierung erfolgt vorzugsweise säurekatalysiert. Das dabei resultierende Glycosid mit dem niedermolekularen Alkohol kann anschließend mit einem erfindungsgemäßen Alkoholgemisch zu den entsprechenden Glycosiden umgesetzt werden. Für diese Reaktion eignen sich im Allgemeinen auch wässrige Saccharidlösungen. Nach einem weiteren geeigneten Verfahren kann das Saccharid zunächst durch Umsetzung mit einem Halogenwasserstoff in das entsprechende O-Acetylhalosaccharid überführt und anschließend mit einem erfindungsgemäßen Alkoholgemisch in Gegenwart säurebindender Verbindungen glycosidiert werden.

Vorzugsweise werden zur Glycosidierung Monosaccharide eingesetzt. Insbesondere werden Hexosen, wie Glucose, Fructose, Galactose, Mannose etc. und Pentosen, wie Arabinose, Xylose, Ribose etc. eingesetzt. Besonders bevorzugt wird Glucose eingesetzt. Die Saccharide können einzeln oder in Form von Gemischen eingesetzt werden. Bei Saccharidgemischen resultieren im Allgemeinen Glycoside mit statistisch verteilten Zuckerresten. Bei mehrfacher Saccharidanlagerung an eine alkoholische Hydroxidgruppe resultieren Polyglycoside der erfindungsgemäßen Alkoholgemische. Auch zu Polyglycosidierung können mehrere Saccharide nacheinander oder als Gemisch eingesetzt werden, so dass die resultierenden funktionalisierten Alkoholgemische die Saccharide in Form von Blöcken oder statistisch verteilt eingebaut enthalten. Es können je nach Reaktionbedingungen, insbesondere Reaktionstemperatur, Furanose- oder Pyranosestrukturen resultieren.

Geeignete Verfahren und Reaktionsbedingungen zur Glycosidierung sind z. B. in Ullmann's Encyclopedia of Industrial Chemistry, 5. Aufl., Bd. A25 (1994), S. 792-793 und den dort zitierten Dokumenten beschrieben.

Die durch Glycosidierung erhaltenen funktionalisierten Alkoholgemische zeigen eine sehr gute Oberflächenaktivität und können als nichtionische Tenside in einer Vielzahl von Anwendungsbereichen vorteilhaft eingesetzt werden.

Die Sulfatierung oder Phosphatierung der zuvor beschriebenen Alkoholgemische oder alkoxylierten Alkoholgemische erfolgt durch Umsetzung mit Schwefelsäure oder Schwefelsäurederivaten zu sauren Alkylsulfaten oder Alkylethersulfaten oder durch Umsetzung mit Phosphorsäure oder oder Phosphorsäurederivaten zu sauren Alkylphosphaten oder Alkyletherphosphaten.

Geeignete Verfahren zur Sulfatierung von Alkoholen sind die üblichen, dem Fachmann bekannten, wie sie z. B. in der US 3,462,525, US 3,420,875 oder US 3,524,864 beschrieben werden, worauf hier in vollem Umfang Bezug genommen wird. Geeignete Verfahren zur Sulfatierung sind auch in Ullmann's Encyclopedia of Industrial Chemistry, 5. Aufl., Bd. A25 (1994), S. 779-783 und der dort zitierten Literatur beschrieben.

Wird zur Sulfatierung der erfindungsgemäßen Alkoholgemische eingesetzt, so ist diese vorzugsweise 75 bis 100 gew.-%ig, insbesondere 85 bis 98 gew.-%ig. Derartige Schwefelsäure ist unter den Bezeichnungen konzentrierte Schwefelsäure und Monohydrat erhältlich.

Gewünschtenfalls kann zur Sulfatierung mit Schwefelsäure ein Lösungs- oder Verdünnungsmittel eingesetzt werden. Geeignete Lösungsmittel sind z. B. solche, die mit Wasser ein Azeotrop bilden, wie z. B. Toluol.

Nach einer geeigneten Ausführungsform zur Herstellung sulfatierter Alkoholgemische wird das Alkoholgemisch in einem Reaktionsgefäß vorgelegt und das Sulfatierungsmittel unter ständigem Durchmischen zugegeben. Zur Erzielung einer möglichst vollständigen Veresterung des Alkoholgemisches beträgt das Molmengenverhältnis von Alkanol zu Sulfatierungsmittel bevorzugt etwa 1:1 bis 1:1,5, insbesondere 1:1 bis 1:1,2. Gewünschtenfalls kann das Sulfatierungsmittel auch in einem molaren Unterschuß eingesetzt werden, z. B. bei der Sulfatierung alkoxylierter Alkoholgemische, wenn Gemische aus nichtionischen und anionischen grenzflächenaktiven Verbindungen hergestellt werden sollen. Die Sulfatierung erfolgt bevorzugt bei einer Temperatur im Bereich von Umgebungstemperatur bis 80 °C, insbesondere 40 bis 75 °C.

Weitere geeignete Sulfatierungsmittel sind z. B. Schwefeltrioxid, Schwefeltrioxid-Komplexe, Lösungen von Schwefeltrioxid in Schwefelsäure (Oleum), Chlorsulfonsäure, Sulfurylchlorid, Amidosulfonsäure etc. Bei Einsatz von Schwefeltrioxid als Sulfatierungsmittel kann die Umsetzung vorteilhaft in einem Fallfilmverdampfer, bevorzugt im Gegenstrom, durchgeführt werden. Dabei kann die Umsetzung diskontinuierlich oder kontinuierlich erfolgen.

Die Aufarbeitung der bei der Sulfatierung entstehenden Reaktionsgemische erfolgt nach üblichen, dem Fachmann bekannten Verfahren. Dazu zählt z. B. die Neutralisierung, Abtrennung gegebenenfalls eingesetzter Lösungsmittel etc.

Die Phosphatierung der zuvor beschriebenen Alkoholgemische und alkoxylierten Alkoholgemische erfolgt im Allgemeinen in analoger Weise zur Sulfatierung.

Geeignete Verfahren zur Phosphatierung von Alkoholen sind die üblichen, dem Fachmann bekannten, wie sie z. B. in Synthesis 1985, S. 449-488 beschrieben werden, worauf hier in vollem Umfang Bezug genommen wird.

Geeignete Phosphatierungsmittel sind z. B. Phosphorsäure, Polyphosphorsäure, Phosphorpentoxid, POCl₃ etc. Bei Einsatz von POCl₃ werden die verbleibenden Säurechloridfunktionen nach der Veresterung hydrolysiert.

Ein weiterer Gegenstand der Erfindung ist die Verwendung der funktionalisierten Alkoholgemische als Tenside, Dispergiermittel, Papierhilfsmittel, Schmutzlösungsmittel, Korrosionsinhibitoren, Hilfsmittel für Dispersionen, Inkrustierungsinhibitoren.

Die Erfindung wird anhand der folgenden, nicht einschränkenden Beispiele näher erläutert.

### Beispiel 1

In einem isotherm betriebenen Reaktor einer Länge von etwa 1,5 m und eines Durchmessers von 30 mm wurde bei 20 bar und 80 °C Raffinat II der nachstehenden Zusammensetzungen an einem heterogenen Katalysator umgesetzt.

| | |
|---|---|
| i-Butan | 3 Gew.-% |
| n-Butan | 15 Gew.-% |
| i-Buten | 2 Gew.-% |
| Buten-1 | 30 Gew.-% |
| Buten-2-trans | 32 Gew.-% |
| Buten-2-cis | 18 Gew.-% |

Als Katalysator diente ein Material, das gemäß der DE-4339713 in Form von Tabletten (5 mm x 5 mm) hergestellt worden war. Die Zusammensetzung in Gew.-% der Aktivkomponenten war: 50 Gew.-% NiO, 12,5 Gew.-% TiO₂, 33,5 Gew.-% SiO₂, 4 Gew.-% Al₂O₃. Der Durchsatz betrug 0,75 kg Raffinat II/(1 Kat x h). Man arbeitete ohne Rückführung von C₄-Kohlenwasserstoffen. Der C₄-Umsatz, bezogen auf die im Raffinat II enthaltenen Butene, betrug 52,0 Gew.-%. Die Selektivität in Gew.-% war wie folgt: C₈: 76,9; C₁₂: 18,4 und C₁₆₊: 4,7. Aus dem erhaltenen Reaktionsgemisch wurde durch fraktionierte Destillation die C₁₂-Olefinfraktion gewonnen.

Zur Charakterisierung der C₁₂-Olefinfraktion wurde eine Probe mit Pd/C in Gegenwart von H₂ hydriert. Laut ¹H-NMR-spektroskopischer Untersuchung weist die gewonnene C₁₂-Paraffin-Fraktion durchschnittlich 4,13 Methylgruppen pro Molekül auf, entsprechend einem ISO-Index von 2,13.

### Beispiel 2

Herstellung von erfindungsgemäßen Tridecanolgemischen durch Hydroformylierung, Hydrierung und Destillation der Dodecengemische.

750 g des gemäß Beispiel 1 hergestellten Dodecengemisches wurden in einem Autoklav mit Hubrührer mit 0,13 Gew.-% Kobalt als Co₂(CO)₈ unter Zusatz von 75 g Wasser bei 185 °C und unter einem Synthesegasdruck von 280 bar und einem Volumenmischungsverhältnis von H₂ zu CO von 60 zu 40 fünf Stunden hydroformyliert; der Verbrauch an Synthesegas, zu erkennen an einem Abfall des Druckes im Autoklaven, wurde durch Nachpressen ergänzt. Nach dem Entspannen des Autoklaven wurde der Reaktionsaustrag nach Zugabe von 10 gew.-%iger wässriger Essigsäure durch Einleiten von Luft oxidativ entkobaltet und die organische Produktphase mit 50 g Raney-Nickel bei 125 °C und bei einem Wasserstoffdruck von 280 bar 10 Stunden hydriert. Durch fraktionierte Destillation des Reaktionsaustrages wurde die Tridecanol-Fraktion von den C₁₂-Paraffinen und von Hochsiedern abgetrennt.

Die OH-Zahl des Tridecanols betrugt 278 mg KOH/g. ¹H-NMR-spektroskopisch wurden 3,27 Methylgruppen/Molekül bestimmt, entsprechend einem Verzweigungsgrad von 2,27.

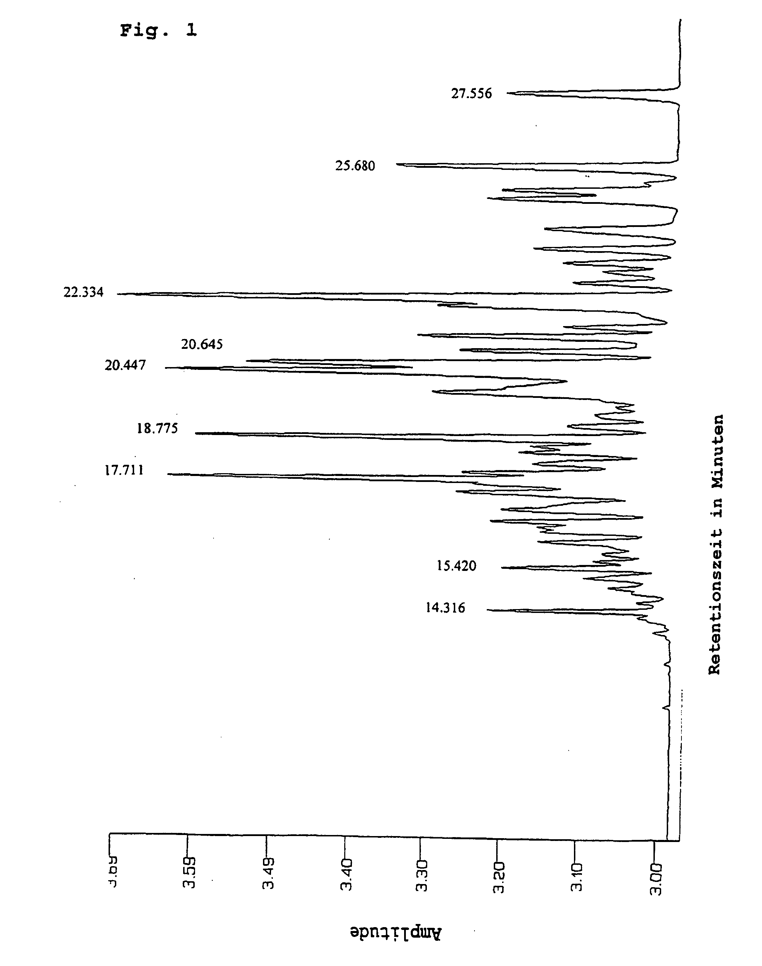

Fig. 1 gibt ein Gaschromatogramm des Tridecanols (als Trimethylsilylether) wieder, das unter folgenden Bedingungen gemessen wurde:

Probenvorbereitung: 3 Tropfen Tridecanol wurden mit 1,5 ml
N-Methyltrimethylsilyltrifluoracetamid 60 min bei 80 °C umgesetzt. Das Injektionsvolumen betrug 1 µl.

Trennbedingungen: Säule; Ultra-1 Hewlett Packard; Länge 50 m, Innendurchmesser 0,33 mm, Filmdicke 0,2 µm; Vordruck 200 mPa; Split 88 ml/min; Temperatur 160 °C (isotherm); Injektortemperatur 250 °C; Detektortemperatur 300 °C; Detektion FID.

### Beispiel 3:

### Herstellung eines Fettalkoholethoxilates mit 7 mol Ethylenoxid

400 g Tridecanol (hergestellt gemäß Beispiel 2) wurden mit 1,5 g NaOH in einen trockenen 2 1-Autoklaven eingefüllt. Der Autoklaveninhalt wurde auf 150 °C erhitzt und 616 g Ethylenoxid unter Druck in den Autoklaven gepresst. Nachdem sich die gesamte Ethylenoxidmenge im Autoklaven befindet, wurde der Autoklav 30 Minuten lang bei 150 °C gehalten. Nach dem Abkühlen wurde der Katalysator mit Schwefelsäure neutralisiert.

Das erhaltene Tensid weist einen Trübungspunkt von 73 °C, gemessen 1 %ig in in 10 %iger Butyldiglycollösung nach DIN 53 917, auf.
Die Oberflächenspannung bei einer Konzentration von 1 g/l beträgt 27,8 mN/m, gemessen nach DIN 53 914.

### Beispiel 4

### Herstellung eines Fettalkoholethoxilates mit 3 mol Ethylenoxid

600 g Tridecanol (hergestellt gemäß Beispiel 2) wurden mit 1,5 g NaOH in einen trockenen 2 l-Autoklaven eingefüllt. Der Autoklaveninhalt wurde auf 150 °C erhitzt und 396 g Ethylenoxid unter Druck in den Autoklaven gepresst. Nachdem sich die gesamte Ethylenoxidmenge im Autoklaven befindet, wurde der Autoklav für 30 Minuten bei 150 °C gehalten. Nach dem Abkühlen wurde der Katalysator mit Schwefelsäure neutralisiert.

Das erhaltene Tensid weist einen Trübungspunkt von 45,5 °C, gemessen 1 %ig in 10 %iger Butyldiglycollösung nach DIN 53 917, auf. Die Oberflächenspannung bei einer Konzentration von 1 g/l beträgt 27,1 mN/m, gemessen nach DIN 53 914.

### Beispiel 5

### Herstellung eines Alkylphosphates

300 g Tridecanol (hergestellt nach Beispiel 2) wurden in einem Rührgefäß unter Stickstoff 60 °C erwärmt und langsam mit 125 g Polyphosphorsäure versetzt, wobei darauf geachtet wurde, dass die Temperatur 65 °C nicht überstieg. Gegen Ende der Zugabe erhöhte man die Temperatur auf 70 °C und rührte eine Stunde bei dieser Temperatur.

Die Oberflächenspannung des erhaltenen Alkylphosphates bei einer Konzentration von 1 g/l beträgt 32,3 mN/m, gemessen nach DIN 53 914.

### Beispiel 6

### Herstellung eines Alkyletherphosphates

560 g des in Beispiel 2 hergestellten Fettalkoholethoxilates wurden in einem Rührgefäß unter Stickstoff auf 60 °C erwärmt und langsam mit 92 g Polyphosphorsäure versetzt, wobei darauf geachtet wurde, dass die Temperatur 65 °C nicht überstieg. Gegen Ende der Zugabe erhöhte man die Temperatur auf 70 °C und rührte eine Stunde bei dieser Temperatur.

Die Oberflächenspannung des erhaltenen Alkyletherphosphates bei einer Konzentration von 1 g/l beträgt 30,8 mN/m, gemessen nach DIN 53 914.

## Patentansprüche

1. Verfahren zur Herstellung eines C₁₃-Alkoholgemischs, bei dem man
a) einen Butene enthaltenden C₄-Kohlenwasserstoffstrom, der weniger als 5 Gew.-%, bezogen auf die Butenfraktion iso-Buten enthält, bei erhöhter Temperatur mit einem Nickel enthaltenden heterogenen Katalysator in Kontakt bringt,
b) aus dem Reaktionsgemisch eine C₁₂-Olefinfraktion isoliert,
c) die C₁₂-Olefinfraktion durch Umsetzung mit Kohlenmonoxid und Wasserstoff in Gegenwart eines Kobalt-Katalysators hydroformyliert und
d) hydriert.

2. Verfahren nach Anspruch 1, wobei die C₁₂-Olefinfraktion einen ISO-Index von 1,9 bis 2,3 aufweist.

3. verfahren nach einem der vorhergehenden Ansprüche, wobei der Butene enthaltende C₄-Kohlenwasserstoffstrom 60 bis 90 Gew.-% Butene und 10 bis 40 Gew.-% Butane enthält.

4. Verfahren nach einem der vorhergehenden Ansprüche, wobei der Oligomerisierungskatalysator Nickeloxid enthält.

5. Verfahren nach Anspruch 4, wobei der Katalysator im Wesentlichen aus NiO, SiO₂, TiO₂ und/oder ZrO₂ sowie gegebenenfalls Al₂O₃ besteht.

6. C₁₃-Alkoholgemisch, dadurch erhältlich, dass man
a) einen Butene enthaltenden C₄-Kohlenwasserstoffstrom, der weniger als 5 Gew.-%, bezogen auf die Butenfraktion iso-Buten enthält, bei erhöhter Temperatur mit einem Nickel enthaltenden heterogenen Katalysator in Kontakt bringt,
b) aus dem Reaktionsgemisch eine C₁₂-Olefinfraktion isoliert,
c) die C₁₂-Olefinfraktion durch Umsetzung mit Kohlenmonoxid und Wasserstoff in Gegenwart eines Kobalt-Katalysators hydroformyliert und
d) hydriert.

7. C₁₃-Alkoholgemisch nach Anspruch 6, das einen Verzweigungsgrad im Bereich von 2,2 bis 2,5 aufweist.

8. Funktionalisiertes Alkoholgemisch, dadurch erhältlich, dass man ein C₁₃-Alkoholgemisch nach Anspruch 6 oder 7 einer Alkoxylierung, Glycosidierung, Sulfatierung, Phosphatierung, Alkoxylierung und nachfolgender Sulfatierung oder Alkoxylierung und nachfolgender Phosphatierung unterwirft.

9. Verwendung des funktionalisierten Alkoholgemisches nach Anspruch 8 als Tensid, Dispergiermittel, Papierhilfsmittel, Schmutzlösungsmittel, Korrosionsinhibitor, Hilfsmittel für Dispersionen oder Inkrustierungsinhibitor.

## Claims

1. A process for preparing a C₁₃-alcohol mixture, which comprises
a) bringing a butene-containing C₄-hydrocarbon stream containing less than 5% by weight, based on the butene fraction, of isobutene into contact with a nickel-containing heterogeneous catalyst at elevated temperature,
b) isolating a C₁₂-olefin fraction from the reaction mixture,
c) hydroformylating the C₁₂-olefin fraction by reaction with carbon monoxide and hydrogen in the presence of a cobalt catalyst and
d) hydrogenating the product from c).

2. A process as claimed in claim 1, wherein the C₁₂-olefin fraction has an ISO index of from 1.9 to 2.3.

3. A process as claimed in either of the preceding claims, wherein the butene-containing C₄-hydrocarbon stream comprises from 60 to 90% by weight of butenes and from 10 to 40% by weight of butanes.

4. A process as claimed in any of the preceding claims, wherein the oligomerization catalyst comprises nickel oxide.

5. A process as claimed in claim 4, wherein the catalyst consists essentially of NiO, SiO₂, TiO₂ and/or ZrO₂ and, if desired, Al₂O₃.

6. A C₁₃-alcohol mixture obtainable by
a) bringing a butene-containing C₄-hydrocarbon stream containing less than 5% by weight, based on the butene fraction, of isobutene into contact with a nickel-containing heterogeneous catalyst at elevated temperature,
b) isolating a C₁₂-olefin fraction from the reaction mixture,
c) hydroformylating the C₁₂-olefin fraction by reaction with carbon monoxide and hydrogen in the presence of a cobalt catalyst and
d) hydrogenating the product from c).

7. A C₁₃-alcohol mixture as claimed in claim 6 which has a degree of branching in the range from 2.2 to 2.5.

8. A functionalized alcohol mixture obtainable by subjecting a C₁₃-alcohol mixture as claimed in claim 6 or 7 to alkoxylation, glycosidation, sulfation, phosphation, alkoxylation and subsequent sulfation or alkoxylation and subsequent phosphation.

9. The use of a functionalized alcohol mixture as claimed in claim 8 as surfactant, dispersant, paper auxiliary, detergent, corrosion inhibitor, auxiliary for dispersions or encrustation inhibitor.

## Revendications

1. Procédé de préparation d'un mélange d'alcool en C₁₃, dans lequel on
a) met en contact un courant d'hydrocarbure en C₄ contenant du butène, qui contient moins de 5 % en poids d'isobutène par rapport à la fraction butène, avec un catalyseur hétérogène contenant du nickel à température élevée,
b) isole du mélange réactionnel une fraction oléfine en C₁₂,
c) hydroformyle la fraction oléfine en C₁₂ par transformation avec du monoxyde de carbone et de l'hydrogène en présence d'un catalyseur du cobalt et
d) hydrogène.

2. Procédé selon la revendication 1, sachant que la fraction oléfine en C₁₂ comporte un indice ISO de 1,9 à 2,3.

3. Procédé selon l'une des revendications précédentes, sachant que le courant d'hydrocarbure en C₄ contenant du butène contient 60 à 90 % en poids de butène et 10 à 40 % en poids de butane.

4. Procédé selon l'une des revendications précédentes, sachant que le catalyseur d'oligomérisation contient de l'oxyde de nickel.

5. Procédé selon la revendication 4, sachant que le catalyseur se compose pour l'essentiel de NiO, SiO₂, TiO₂ et/ou ZrO₂ ainsi que le cas échéant Al₂O₃.

6. Mélange d'alcool en C₁₃, que l'on peut obtenir par le fait que l'on
a) met en contact un courant d'hydrocarbure en C₄ contenant du butène, qui contient moins de 5 % en poids d'isobutène par rapport à la fraction butène, avec un catalyseur hétérogène contenant du nickel à température élevée,
b) isole du mélange réactionnel une fraction oléfine en C₁₂,
c) hydroformyle la fraction oléfine en C₁₂ par transformation avec du monoxyde de carbone et de l'hydrogène en présence d'un catalyseur du cobalt et
d) hydrogène.

7. Mélange d'alcool en C₁₃ selon la revendication 6, qui comporte un degré de ramification dans le domaine de 2,2 à 2,5.

8. Mélange d'alcool fonctionnalisé, que l'on peut obtenir en soumettant un mélange d'alcool en C₁₃ selon la revendication 6 ou 7 à une alcoxylation, une glycosidation, une sulfatation, une phosphatation, une alcoxylation et sulfatation ultérieure ou une alcoxylation et phosphatation ultérieure.

9. Utilisation du mélange d'alcool fonctionnalisé selon la revendication 8 en tant qu'agent tensioactif, agent de dispersion, agent correcteur du papier, agent dissolvant de salissure, inhibiteur de corrosion, agent correcteur pour les dispersions ou inhibiteur d'incrustation.
